(19) Europäisches Patentamt
European Patent Office
Office européen des brevets

(11) Veröffentlichungsnummer: **0 229 974**
B1

(12) # EUROPÄISCHE PATENTSCHRIFT

(45) Veröffentlichungstag der Patentschrift: **14.11.90**

(51) Int. Cl.⁵: **C07C 69/734**, A01N 47/36

(21) Anmeldenummer: **86117118.9**

(22) Anmeldetag: **09.12.86**

(54) **Acrylsäurederivate und Fungizide, die diese Verbindungen enthalten.**

(30) Priorität: **20.12.85 DE 3545318**

(43) Veröffentlichungstag der Anmeldung: **29.07.87 Patentblatt 87/31**

(45) Bekanntmachung des Hinweises auf die Patenterteilung: **14.11.90 Patentblatt 90/46**

(84) Benannte Vertragsstaaten: **AT BE CH DE ES FR GB GR IT LI NL SE**

(56) Entgegenhaltungen:
EP-A- 0 178 826
US-A- 3 639 445

(73) Patentinhaber: **BASF Aktiengesellschaft, Carl-Bosch-Strasse 38, D-6700 Ludwigshafen(DE)**

(72) Erfinder: **Schirmer, Ulrich, Dr., Berghalde 79, D-6900 Heidelberg(DE)**
Erfinder: **Karbach, Stefan, Dr., Sperlinggasse 3, D-6700 Ludwigshafen(DE)**
Erfinder: **Pommer, Ernst-Heinrich, Dr., Berliner Platz 7, D-6703 Limburgerhof(DE)**
Erfinder: **Ammermann, Eberhard, Dr., Sachsenstrasse 3, D-6700 Ludwigshafen(DE)**
Erfinder: **Steglich, Wolfgang, Prof. Dr., Hobsweg 77, D-5300 Bonn-Roettgen(DE)**
Erfinder: **Schwalge, Barbara Agnes Maria, Tannenweg 9, D-5204 Lohmar 1(DE)**
Erfinder: **Anke, Timm, Prof. Dr., Theodor-Heuss-Strasse 17, D-6750 Kaiserslautern(DE)**

EP 0 229 974 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Acrylsäurederivate und Fungizide, welche diese Verbindungen enthalten.

Es ist bekannt, N-Trichlormethylthio-tetrahydrophthalimid als Fungizid in der Landwirtschaft sowie im Obst- und Gartenbau einzusetzen (Chem. Week, June 21, 1972, Seite 46). Das bekannte Mittel ist jedoch nur vor der Infektion verwendbar und seine Wirkung genügt bei niedrigen Aufwandmengen den Anforderungen der Praxis nicht.

Es wurde nun gefunden, daß neue Acrylsäurederivate der Formel

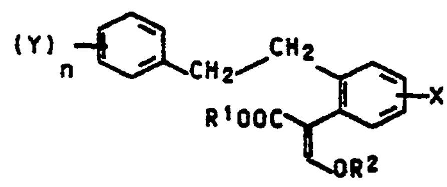

in der $R^1$ und $R^2$ unabhängig voneinander $C_1$–$C_8$-Alkyl bedeuten,
X Wasserstoff, Halogen, $C_1$–$C_4$-Alkoxy, Trifluormethyl, Cyano oder Nitro bedeutet,
Y Wasserstoff, Alkyl, Halogenalkyl, Alkoxyalkyl, Cycloalkyl, Aralkyl, Aryl, Aryloxy, Halogen, eine gegebenenfalls substituierte an den Benzolrest annellierte $C_4H_4$-Kette, Alkoxy, Halogenalkoxy, $NO_2$, Alkylthio, Thiocyanato, Cyano,

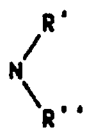 , NHCOR', 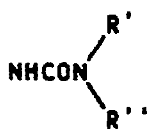 , COOR', 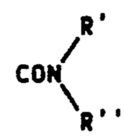 , OSO₂R', SO₂R', COR',

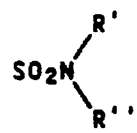

bedeutet, wobei R′ und R″ jeweils unabhängig voneinander Wasserstoff, Alkyl, Alkoxy, Alkylthio, Cycloalkyl oder gegebenenfalls durch Alkyl, Halogen oder Alkoxy substituiertes Phenyl bedeuten und n die Zahlen 1 bis 4 bedeutet, außer den Verbindungen, in denen $R^1$ und $R^2$ Methyl, X Wasserstoff und Y Wasserstoff, Halogen, Methyl, Ethyl, iso-Propyl, tertiär-Butyl, Methoxy, Trifluormethoxy, Nitro, Amino, Phenyl Carboxyl, Methoxycarbonyl, Cyano, Acetylamino bedeutet,
eine ausgezeichnete fungizide Wirkung haben.

Die in der allgemeinen Formel angeführten Reste können beispielsweise folgende Bedeutung haben:
$R^1$ bzw. $R^2$ gegebenenfalls verzweigtes $C_1$–$C_8$-Alkyl (z.B. Methyl, Ethyl, Isopropyl, n-Propyl, n-Butyl, sec.-Butyl, tert.-Butyl, iso-Butyl, sec.-Pentyl, n-Hexyl, α-Ethyl-n-hexyl, n-Octyl),
X Wasserstoff, Halogen (z.B. Fluor, Chlor, Brom), $C_1$–$C_4$-Alkoxy (z.B. Methoxy, n-Butoxy) Trifluormethyl, Cyano oder $NO_2$.
Y Wasserstoff, $C_1$–$C_{12}$-Alkyl (z.B. Methyl, Ethyl, t-Butyl, Dodecyl), Halogen-$C_1$–$C_4$-alkyl, (z.B. Trifluormethyl), $C_1$–$C_4$-Alkoxy-$C_1$–$C_4$-alkyl (z.B. Methoxymethyl), $C_5$–$C_8$-Cycloalkyl (z.B. Cyclohexyl), Aralkyl (z.B. Benzyl), Aryl (z.B. Phenyl), Aryloxy (z.B. Phenoxy), Halogen (z.B. Fluor, Chlor, Brom oder Jod), eine gegebenenfalls substituierte $C_4H_4$-Kette, die mit dem Benzolring zu einem gegebenenfalls substituierten Naphthylring kondensiert ist, $C_1$–$C_8$-Alkoxy (z.B. Isopropoxy, Hexyloxy), Halogen-$C_1$–$C_4$-alkoxy (z.B. 1,1,2,2-Tetrafluorethoxy), $C_1$–$C_4$-Alkylthio (z.B. Methylthio), Thiocyanato, Cyano, $NO_2$,

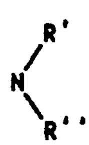 , 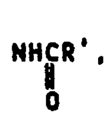 , 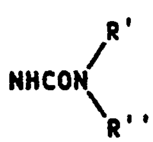 , COOR', 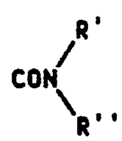 , SO₂R', COR', OSO₂R',

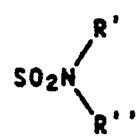

wobei R′ und R″ jeweils unabhängig voneinander Wasserstoff, $C_1$–$C_4$-Alkyl (z.B. Methyl, Ethyl), $C_1$–$C_4$-Alkoxy (z.B. Methoxy, tert.-Butoxy), $C_1$–$C_4$-Alkylthio (z.B. Methylthio), $C_5$–$C_8$-Cycloalkyl (z.B. Cyclohexyl) oder gegebenenfalls durch $C_1$–$C_4$-Alkyl, Halogen oder $C_1$–$C_4$-Alkoxy substituiertes Phenyl (z.B. Phenyl, 3-Chlorphenyl, 4-Methylphenyl, 3-Methoxyphenyl) bedeuten.

Die neuen Verbindungen können beispielsweise nach folgendem Verfahren hergestellt werden: 2-Methylphenylessigsäureester der allgemeinen Formel

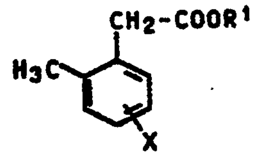

werden nach Wislicenus (Liebigs Annalen 424, 215 (1921) und Liebigs Annalen 413, 206 (1917)) mit Ameisensäuremethylester und Natriumhydrid in einem inerten Lösungsmittel zur Reaktion gebracht. Die so erhaltenen Verbindungen der allgemeinen Formel

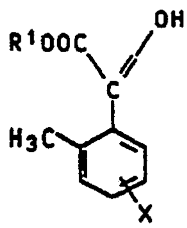

werden mit einem Alkylierungsmittel in Gegenwart einer Base in einem Lösungsmittel (z.B. Aceton) zu α-(2-Methylphenyl)-β-alkoxyacrylsäureestern umgesetzt.

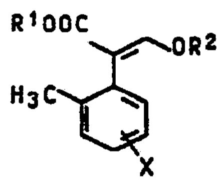

in denen $R^1$, $R^2$ und X die oben genannten Bedeutungen haben.

Die Bromierung dieser Verbindungen mit N-Bromsuccinimid (Horner, Winkelmann, Angew. Chemie 71, 349 (1959) führt zu α-(2-Brommethylphenyl)-β-alkoxy-acrylsäureestern, die mit Phosphorigsäuretrialkylestern zu Phosphonsäureestern der allgemeinen Formel reagieren:

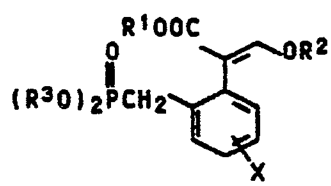

in der $R^1$, $R^2$ und X die oben genannten Bedeutungen haben und $R^3$ für $C_1$–$C_8$-Alkyl steht (Houben-Weyl, Methoden der organischen Chemie 12/1, 433 ff (1963)).

Die oben genannten Phosphonsäureester werden mit gegebenenfalls substituierten Benzaldehyden zu Stilbenderivaten umgesetzt:

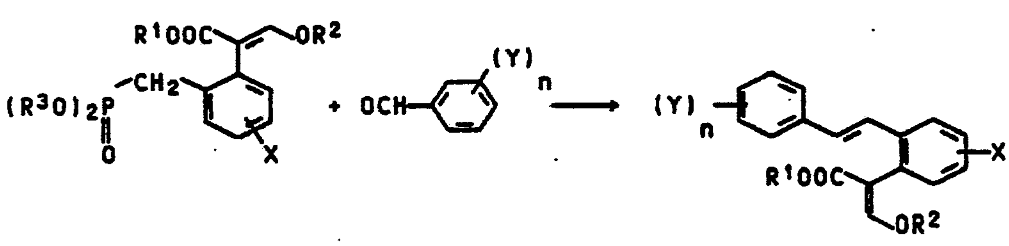

(vgl. Wadsworth, Emmons, JACS. 83, 1732 (1961)).

Die so erhaltenen Stilbenderivate lassen sich entweder katalytisch mit Wasserstoff (vgl. Houben-Weyl, Methoden der organischen Chemie V/2b, 264–267 (1981) oder mit Diimin (vgl. Houben-Weyl, Methoden der organischen Chemie IV/1c, 580 und E.E. van Tamelen, R.S. Dewey, M.F. Lease & W.H. Pirkle, JACS. 83, 4302 (1961) selektiv zu den neuen Acrylsäurederivaten reduzieren:

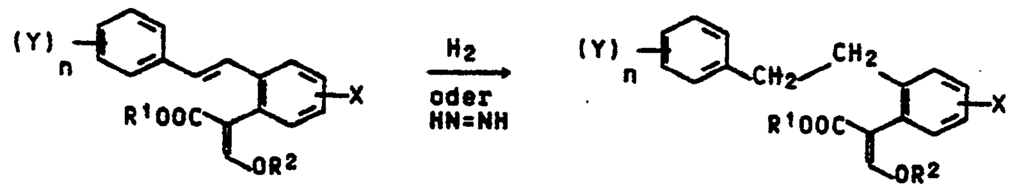


Die nachstehenden Vorschriften erläutern die Synthese der Ausgangsverbindungen:

Vorschrift A

α-(2-Methylphenyl)-b-methoxy-acrylsäuremethylester

16,5 g 2-Methylphenylessigsäuremethylester werden in 10 ml Ameisensäuremethylester gelöst und langsam zu einer Suspension aus 3 g Natriumhydrid in 150 ml abs. Ether getropft. Nach 4 h Rückfluß wird mit verdünnter HCl angesäuert, die org. Phase abgetrennt, mit Wasser gewaschen, über $MgSO_4$ getrocknet und eingeengt. Man erhält 13,8 g eines hellgelben Öles (α-Formyl-(2-methlphenyl)essigsäuremethylester), das zusammen mit 5,8 ml Dimethylsulfat, 10,9 g Kaliumcarbonat und 70 ml Aceton 1 h am Rückfluß gekocht wird. Nach dem Filtrieren und Einengen wird in Ether aufgenommen, dann mit verdünntem wäßrigem Ammoniak und mehrfach mit Wasser gewaschen. Nach Abziehen des Ethers erhält man 11,3 g rohen α-(2-Methylphenyl)-β-methoxyacrylsäuremethylester ($Kp_{0,05}$ 102–108°C).

| | | | |
|---|---|---|---|
| NMR in $CDCl_3$: | 7,53 | s | 1H |
| | 7,16–7,36 | bs | 4H |
| | 3,64 | s | 3H |
| | 3,73 | s | 3H |
| | 2,16 | s | 3H |

Vorschrift B

α-(2-Brommethylphenyl)-β-methoxyacrylsäuremethylester

20,6 g des nach Vorschrift A erhaltenen α-(2-Methylphenyl)-β-methoxyacrylsäuremethylesters, 17,65 g N-Bromsuccinimid, 0,2 g Azodiisobutyronitril und 150 ml $CCl_4$ werden langsam auf 90°C erhitzt. Man hält bei dieser Temperatur bis alles Succinimid auf dem Lösungsmittel schwimmt. Nach Filtration wird eingeengt, das verbleibende Öl in etwa 5 ml Aceton gelöst und mit n-Hexan zur Kristallisation gebracht. Man erhält 27,5 g farblose Kristalle vom Fp. 86–87°C.

Vorschrift C

2-(β-Methoxy-α-methoxycarbonyl-vinyl)benzylphosphonsäuredimethylester

28,5 g α-(2-Brommethylphenyl)-β-methoxyacrylsäuremethylester werden mit 11,8 ml Phosphorigsäuretrimethylester und 6,5 ml Toluol eine Stunde am Rückfluß gekocht. Das Reaktionsgemisch wird vorsichtig im Vakuum eingeengt, das verbleibende Öl wird nach Lösen in 5 ml Ether mit n-Hexan zur Kristallisation gebracht. Man erhält 27,3 g farblose Kristalle vom Fp. 94°C.

Vorschrift D

2-(β-Methoxy-α-methoxycarbonylvinyl)stilben

3,14 g 2-(β-Methoxy-α-methoxycarbonylvinyl)benzylphopshonsäuredimethylester werden gelöst in 7 ml abs. Tetrahydrofuran (THF) bei 0°C zu 0,3 g Natriumhydrid in 5 ml abs. THF getropft. Nach 20 bis 30 Min. versetzt man mit 1,1 ml Benzaldehyd, läßt auf 20°C erwärmen und kocht dann 5 h am Rückfluß. Nach dem Abkühlen wird eingeengt und mit 15 ml Wasser und 70 ml Ether versetzt. Die organische Phase wird dann 3× mit je 15 ml 10% (Gew.%) wäßriger $NaHCO_3$-Lösung sowie 3× mit gesättigter NaCl-Lösung ausgeschüttelt. Nach dem Trocknen über $MgSO_4$ wird eingeengt und schließlich aus Chloroform/Hexan umkristallisiert. Man erhält 1,7g farblose Kristalle vom Fp. 107–109°C 2-(β-Methoxy-α-methoxy-carbonylvinyl)stilben.

In entsprechender Weise lassen sich folgende Verbindungen herstellen:

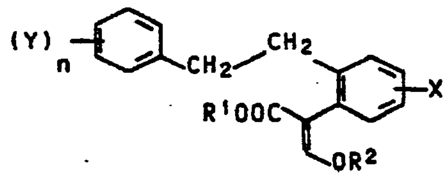

| Nr. | $R^1$ | $R^2$ | X | $(Y)_n$ | Fp °C/NMR |
|---|---|---|---|---|---|
| 2 | $C_2H_5$ | $CH_3$ | H | H | |
| 3 | $i\text{-}C_3H_7$ | $CH_3$ | H | H | |
| 4 | $nC_6H_{13}$ | $CH_3$ | H | H | |
| 5 | $n\text{-}C_4H_9$ | $CH_3$ | H | H | |
| 6 | $n\text{-}C_3H_7$ | $CH_3$ | H | H | |
| 7 | $s\text{-}C_4H_9$ | $CH_3$ | H | H | |
| 8 | $CH_3$ | $C_2H_5$ | H | H | |
| 9 | $CH_3$ | $i\text{-}C_3H_7$ | H | H | |
| 10 | $CH_3$ | $n\text{-}C_6H_{13}$ | H | H | |
| 11 | $CH_3$ | $n\text{-}C_3H_7$ | H | H | |
| 12 | $CH_3$ | $s\text{-}C_4H_9$ | H | H | |
| 14 | $CH_3$ | $CH_3$ | 3-Cl | H | |
| 15 | $CH_3$ | $CH_3$ | 4-Cl | H | |
| 16 | $CH_3$ | $CH_3$ | 5-Cl | H | |
| 17 | $CH_3$ | $CH_3$ | 6-Cl | H | |
| 18 | $CH_3$ | $CH_3$ | $5\text{-}OCH_3$ | H | |
| 19 | $CH_3$ | $CH_3$ | $5\text{-}CF_3$ | H | |
| 20 | $CH_3$ | $CH_3$ | 5-CN | H | |
| 21 | $CH_3$ | $CH_3$ | $5\text{-}NO_2$ | $4\text{-}CH_3$ | |
| 22 | $CH_3$ | $CH_3$ | $6\text{-}NO_2$ | H | |

| Nr. | $R^1$ | $R^2$ | X | $(Y)_n$ | Fp °C/NMR |
|---|---|---|---|---|---|
| 36 | $CH_3$ | $CH_3$ | H | 3-$CF_3$ | 48-50 |
| 37 | $CH_3$ | $CH_3$ | H | 4-$CF_3$ | 83-87 |
| 39 | $CH_3$ | $CH_3$ | H | 3-Phenoxy | Öl |
| 40 | $CH_3$ | $CH_3$ | H | 4-Phenoxy | |
| 44 | $CH_3$ | $CH_3$ | H | 4-O(t)-$C_4H_9$ | |
| 45 | $CH_3$ | $CH_3$ | H | 4-O(n)-$C_4H_9$ | |
| 46 | $CH_3$ | $CH_3$ | H | 4-$CH_2OCH_3$ | |
| 48 | $CH_3$ | $CH_3$ | H | 2,3 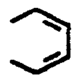 | Öl |
| 49 | $CH_3$ | $CH_3$ | H | 3,4 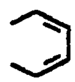 | 92-93 |
| 50 | $CH_3$ | $CH_3$ | H | 4-$OCHF_2$ | |
| 51 | $CH_3$ | $CH_3$ | H | 3-$OCF_2CHF_2$ | |
| 52 | $CH_3$ | $CH_3$ | H | 4-$SCH_3$ | |
| 55 | $CH_3$ | $CH_3$ | H | 4-SCN | |
| 56 | $CH_3$ | $CH_3$ | H | 4-$N(CH_3)_2$ | |
| 58 | $CH_3$ | $CH_3$ | H | 3-$NHCOOCH_3$ | |
| 59 | $CH_3$ | $CH_3$ | H | 4-$NHCON(CH_3)_2$ | |
| 61 | $CH_3$ | $CH_3$ | H | 4-$CONHCH_3$ | |
| 62 | $CH_3$ | $CH_3$ | H | 4-$SO_2CH_3$ | |
| 63 | $CH_3$ | $CH_3$ | H | 4-Phenylsulfonyl | |

| Nr. | $R^1$ | $R^2$ | X | $(Y)_n$ | Fp °C/NMR |
|---|---|---|---|---|---|
| 64 | $CH_3$ | $CH_3$ | H | 3-$COCH_3$ | |
| 65 | $CH_3$ | $CH_3$ | H | 4-$OSO_2CH_3$ | |
| 66 | $CH_3$ | $CH_3$ | H | 4-$SO_2N(CH_3)_2$ | |
| 67 | $CH_3$ | $CH_3$ | H | 4-NHCONH— 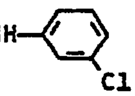 | |
| 68 | $CH_3$ | $CH_3$ | H | 4-Benzoyl | |
| 81 | $CH_3$ | $CH_3$ | H | 4-$N(C_2H_5)_2$ | |
| 84 | $CH_3$ | $CH_3$ | H | 3-Benzyloxy | Öl |
| 86 | $CH_3$ | $CH_3$ | H | 4-$O(n)C_6H_{13}$ | |
| 90 | $CH_3$ | $CH_3$ | H | 2-$OCF_2CHF_2$ | |
| 92 | $C_2H_5$ | $CH_3$ | H | 3-Cl | |
| 93 | $C_2H_5$ | $CH_3$ | H | 4-Cl | |
| 94 | $C_2H_5$ | $CH_3$ | H | 3,5$Cl_2$ | |
| 95 | $C_2H_5$ | $CH_3$ | H | 4-F | |
| 96 | $C_2H_5$ | $CH_3$ | H | 4-Br | |
| 97 | $C_2H_5$ | $CH_3$ | H | 4-$CH_3$ | |
| 98 | $C_2H_5$ | $CH_3$ | H | 3,4-$(CH_3)_2$ | |
| 99 | $C_2H_5$ | $CH_3$ | H | 4-$OCH_3$ | |
| 100 | $C_2H_5$ | $CH_3$ | H | 3,4,5-$(OCH_3)_3$ | |
| 101 | $C_2H_5$ | $CH_3$ | H | 3-$CH_3$ | |
| 102 | $C_2H_5$ | $CH_3$ | H | 4-$(t)C_4H_9$ | |
| 103 | $C_2H_5$ | $CH_3$ | H | 2$CH_3$ | |
| 104 | $CH_3$ | $C_2H_5$ | H | 4-Cl | |
| 105 | $CH_3$ | $C_2H_5$ | H | 4-F | |
| 106 | $CH_3$ | $C_2H_5$ | H | 4-$CH_3$ | |
| 107 | $CH_3$ | $C_2H_5$ | H | 4-$OCH_3$ | |
| 108 | $CH_3$ | $C_2H_5$ | H | 4-$NO_2$ | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten, Phycomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse – wie Gurken, Bohnen und Kürbisgewächse –.

Die neuen Verbindungen sind insbesondere geeignet zur Bekämpfung folgender Pflanzenkrankheiten:

Erysiphe graminis (echter Mehltau) in Getreide,

Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Pyrenophora teres an Gerste,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Alternaria solani an Kartoffeln, Tomaten,
Plasmopara viticola an Reben sowie Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff. Die Aufwandmengen liegen je nach Art des gewünschten Effektes zwischen 0,05 und 3 kg Wirkstoff oder mehr je ha.

Die neuen Verbindungen können auch im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile lösemittelhaltiger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Darüber hinaus haben einige der neuen Verbindungen auch eine sehr gute Wirksamkeit gegen humanpathogene Pilze, insbesondere gegen Trichophyton mentagrophytes und Candida albicans.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Die erfindungsgemäßen Mittel können auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fälen eine Vergrößerung des fungiziden Wirkungsspektrums.

**Patentansprüche**

1. Acrylsäurederivate der allgemeinen Formel

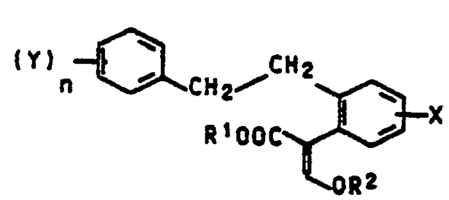

in der $R^1$ und $R^2$ unabhängig voneinander $C_1$–$C_8$-Alkyl bedeuten,
X Wasserstoff, Halogen, $C_1$–$C_4$-Alkoxy, Trifluormethyl, Cyano oder Nitro bedeutet,

Y Wasserstoff, Alkyl, Halogenalkyl, Alkoxyalkyl, Cycloalkyl, Aralkyl, Aryl, Aryloxy, Halogen, eine gegebenenfalls substituierte an den Benzolrest annellierte $C_4H_4$-Kette, Alkoxy, Halogenalkoxy, Alkylthio, Thiocyanato, Cyano, $NO_2$,

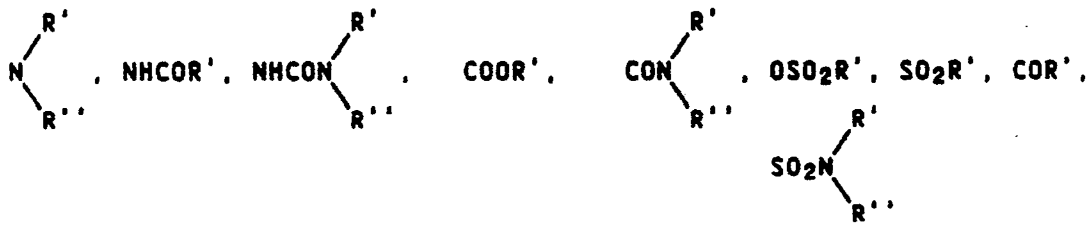

bedeutet, wobei R′ und R″ jeweils unabhängig voneinander Wasserstoff, Alkyl, Alkoxy, Alkylthio, Cycloalkyl oder gegebenenfalls durch Alkyl, Halogen oder Alkoxy substituiertes Phenyl bedeuten und n die Zahlen 1 bis 4 bedeutet,
außer den Verbindungen, in denen $R^1$ und $R^2$ Methyl, X Wasserstoff und Y Wasserstoff, Halogen, Methyl, Ethyl, iso-Propyl, tertiär-Butyl, Methoxy, Trifluormethoxy, Nitro, Amino, Phenyl, Carboxyl, Methoxycarbonyl, Cyano, Acetylamino bedeutet.

2. Fungizid, enthaltend einen Trägerstoff und eine fungizid wirksame Menge einer Verbindung der allgemeinen Formel

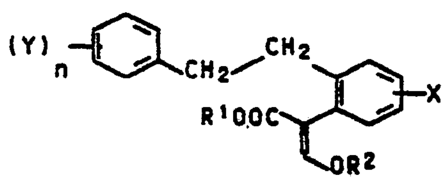

in der $R^1$ und $R^2$ unabhängig voneinander $C_1$–$C_8$-Alkyl bedeuten,
X Wasserstoff, Halogen, $C_1$–$C_4$-Alkoxy, Trifluormethyl, Cyano oder Nitro bedeutet,
Y Wasserstoff, Alkyl, Halogenalkyl, Alkoxyalkyl, Cycloalkyl, Aralkyl, Aryl, Aryloxy, Halogen, eine gegebenenfalls substituierte an den Benzolrest annellierte $C_4H_4$-Kette, Alkoxy, Halogenalkoxy, Alkylthio, Thiocyanato, Cyano, $NO_2$,

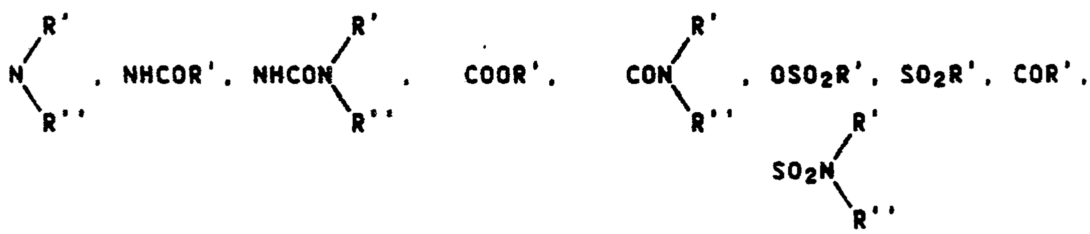

bedeutet, wobei R′ und R″ jeweils unabhängig voneinander Wasserstoff, Alkyl, Alkoxy, Alkylthio, Cycloalkyl oder gegebenenfalls durch Alkyl, Halogen oder Alkoxy substituiertes Phenyl bedeuten und n die Zahlen 1 bis 4 bedeutet,
außer den Verbindungen, in denen $R^1$ und $R^2$ Methyl, X Wasserstoff und Y Wasserstoff, Halogen, Methyl, Ethyl, iso-Propyl, tertiär-Butyl, Methoxy, Trifluormethoxy, Nitro, Amino, Phenyl, Carboxyl, Methoxycarbonyl, Cyano, Acetylamino bedeutet.

3. Verfahren zur Bekämpfung von Pilzen, dadurch gekennzeichnet, daß man die Pilze oder die von Pilzbefall bedrohten Materialien, Pflanzen, Saatgüter oder den Boden mit einer fungizid wirksamen Menge einer Verbindung der allgemeinen Formel

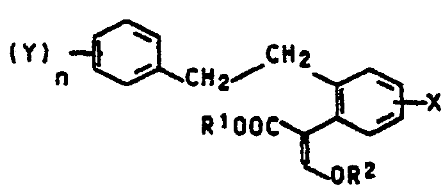

behandelt,

in der $R^1$ und $R^2$ unabhängig voneinander $C_1$–$C_8$-Alkyl bedeuten,
X Wasserstoff, Halogen, $C_1$–$C_4$-Alkoxy, Trifluormethyl, Cyano oder Nitro bedeutet,
Y Wasserstoff, Alkyl, Halogenalkyl, Alkoxyalkyl, Cycloalkyl, Aralkyl, Aryl, Aryloxy, Halogen, eine gegebenenfalls substituierte an den Benzolrest annellierte $C_4H_4$-Kette, Alkoxy, Halogenalkoxy, Alkylthio, Thiocyanato, Cyano, $NO_2$,

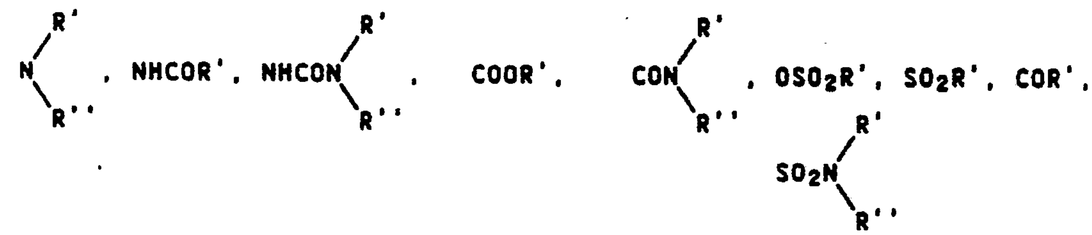
, NHCOR', , COOR', , $OSO_2R'$, $SO_2R'$, COR',

bedeutet, wobei R′ und R″ jeweils unabhängig voneinander Wasserstoff, Alkyl, Alkoxy, Alkylthio, Cycloalkyl oder gegebenenfalls durch Alkyl, Halogen oder Alkoxy substituiertes Phenyl bedeuten und n die Zahlen 1 bis 4 bedeutet,
außer den Verbindungen, in denen $R^1$ und $R^2$ Methyl, X Wasserstoff und Y Wasserstoff, Halogen, Methyl, Ethyl, iso-Propyl, tertiär-Butyl, Methoxy, Trifluormethoxy, Nitro, Amino, Phenyl, Carboxyl, Methoxycarbonyl, Cyano, Acetylamino bedeutet.

## Claims

1. An acrylic acid derivative of the formula

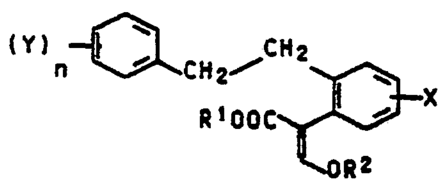

where $R^1$ and $R^2$ independently of one another are each $C_1$–$C_8$-alkyl, X is hydrogen, halogen, $C_1$–$C_4$-alkoxy, trifluoromethyl, cyano or nitro, Y is hydrogen, alkyl, haloalkyl, alkoxyalkyl, cycloalkyl, aralkyl, aryl, aryloxy, halogen, an unsubstituted or substituted $C_4H_4$ chain which is fused to the benzene radical, alkoxy, haloalkoxy, alkylthio, thiocyanato, cyano, $NO_2$,

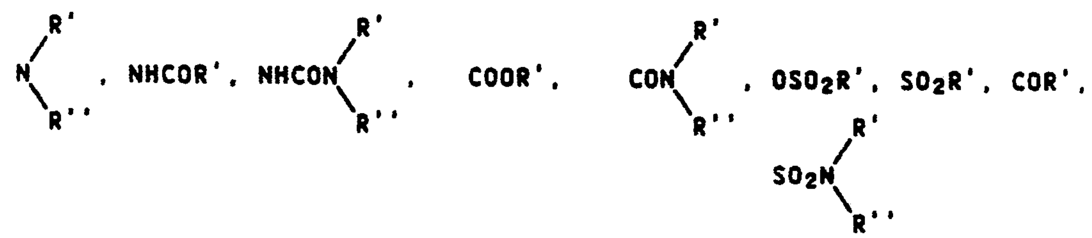
, NHCOR', , COOR', , $OSO_2R'$, $SO_2R'$, COR',

where
R′ and R″ independently of one another are each hydrogen, alkyl, alkoxy, alkylthio or cycloalkyl or are each phenyl which is unsubstituted or substituted by alkyl, halogen or alkoxy, and n is from 1 to 4,
apart from the compounds where $R^1$ and $R^2$ are each methyl, X is hydrogen and Y is hydrogen, halogen, methyl, ethyl, isopropyl, tert-butyl, methoxy, trifluoromethoxy, nitro, amino, phenyl, carboxyl, methoxycarbonyl, cyano or acetylamino.

2. A fungicide containing a carrier and a compound of the formula

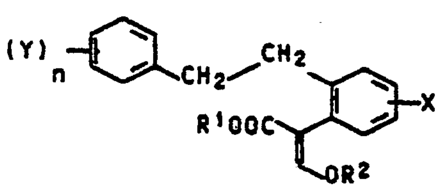

where $R^1$ and $R^2$ independently of one another are each $C_1$–$C_8$-alkyl, X is hydrogen, halogen, $C_1$–$C_4$-alkoxy, trifluoromethyl, cyano or nitro, Y is hydrogen, alkyl, haloalkyl, alkoxyalkyl, cycloalkyl, aralkyl, aryl, aryloxy, halogen, an unsubstituted or substituted $C_4H_4$ chain which is fused to the benzene radical, alkoxy, haloalkoxy, alkylthio, thiocyanato, cyano, $NO_2$,

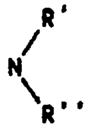 , NHCOR', 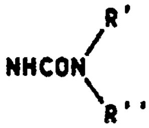 , COOR', 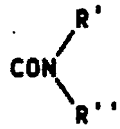 , $OSO_2R'$, $SO_2R'$, COR', 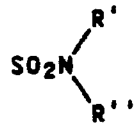

where
R′ and R″ independently of one another are each hydrogen, alkyl, alkoxy, alkylthio or cycloalkyl or are each phenyl which is unsubstituted or substituted by alkyl, halogen or alkoxy, and n is from 1 to 4, apart from the compounds where $R^1$ and $R^2$ are each methyl, X is hydrogen and Y is hydrogen, halogen, methyl, ethyl, isopropyl, tert-butyl, methoxy, trifluoromethoxy, nitro, amino, phenyl, carboxyl, methoxycarbonyl, cyano or acetylamino.

3. A process for combating fungi, wherein the fungi or the materials, plants, seed or the soil under threat of fungal attack are treated with a fungicidally effective amount of a compound of the formula

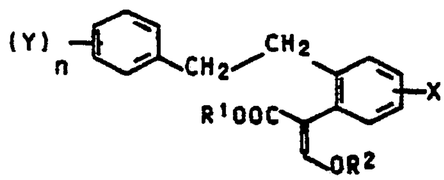

where $R^1$ and $R^2$ independently of one another are each $C_1$–$C_8$-alkyl, X is hydrogen, halogen, $C_1$–$C_4$-alkoxy, trifluoromethyl, cyano or nitro, Y is hydrogen, alkyl, haloalkyl, alkoxyalkyl, cycloalkyl, aralkyl, aryl, aryloxy, halogen, an unsubstituted or substituted $C_4H_4$ chain which is fused to the benzene radical, alkoxy, haloalkoxy, alkylthio, thiocyanato, cyano, $NO_2$,

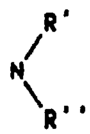 , NHCOR', 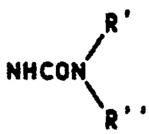 , COOR', 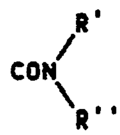 , $OSO_2R'$, $SO_2R'$, COR', 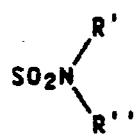

where
R′ and R″ independently of one another are each hydrogen, alkyl, alkoxy, alkylthio or cycloalkyl or are each phenyl which is unsubstituted or substituted by alkyl, halogen or alkoxy, and n is from 1 to 4, apart from the compounds where $R^1$ and $R^2$ are each methyl, X is hydrogen and Y is hydrogen, halogen, methyl, ethyl, isopropyl, tert-butyl, methoxy, trifluoromethoxy, nitro, amino, phenyl, carboxyl, methoxycarbonyl, cyano or acteylamino.

## Revendications

1. Dérivé de l'acide acrylique de la formule générale

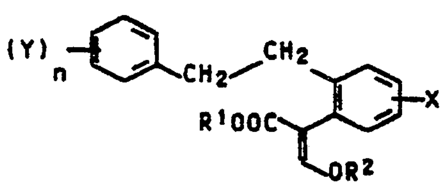

dans laquelle $R^1$ et $R^2$ représentent, indépendamment l'un de l'autre, un radical alkyle en $C_1$–$C_8$, X repré-

sente un atome d'hydrogène, un atome d'halogène, un radical alcoxy en $C_1$–$C_4$, trifluorométhyle, cyano ou nitro, Y représente un atome d'hydrogène, un radical alkyle, halogénalkyle, alcoxyalkyle, cycloalkyle, aralkyle, aryle, aryloxy, un atome d'halogène, une chaîne $C_4H_4$ éventuellement substituée, condensée avec un reste de benzène, un radical alcoxy, halogénalcoxy, alkylthio, thiocyanato, cyano, $NO_2$

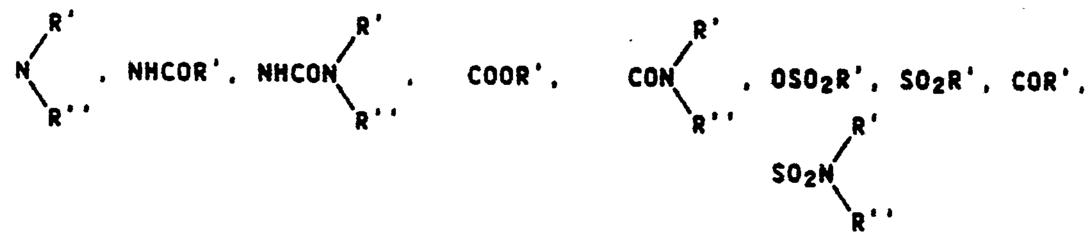

où $R^1$ et $R^2$ représentent, chacun indépendamment de l'autre, un atome d'hydrogène, un radical alkyle, alcoxy, alkylthio, cycloalkyle ou phényle à substitution alkylique, halogénée ou alcoxylique éventuelle et n représente les nombres 1 à 4,

à l'exception des composés dans lesquels $R^1$ et $R^2$ représentent le radical méthyle, X représente l'atome d'hydrogène et Y représente un atome d'hydrogène, d'halogène, un radical méthyle, éthyle, isopropyle, butyle tertiaire, méthoxy, trifluorométhoxy, nitro, amino, phényle, carboxyle, méthoxycarbonyle, cyano, acétylamino.

2. Fongicide qui contient un support ou véhicule et une quantité efficace du point de vue fongicide d'un composé de la formule générale

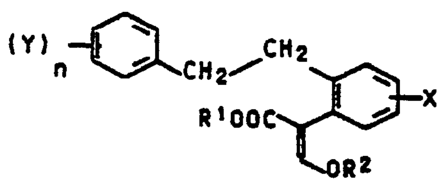

dans laquelle $R^1$ et $R^2$ représentent, indépendamment l'un de l'autre, un radical alkyle en $C_1$–$C_8$, X représente un atome d'hydrogène, un atome d'halogène, un radical alcoxy en $C_1$–$C_4$, trifluorométhyle, cyano ou nitro,

Y représente un atome d'hydrogène, un radical alkyle, halogénalkyle, alcoxyalkyle, cycloalkyle, aralkyle, aryle, aryloxy, un atome d'halogène, une chaîne $C_4H_4$ éventuellement substituée, condensée avec un reste de benzène, un radical alcoxy, halogénalcoxy, alkylthio, thiocyanato, cyano, $NO_2$

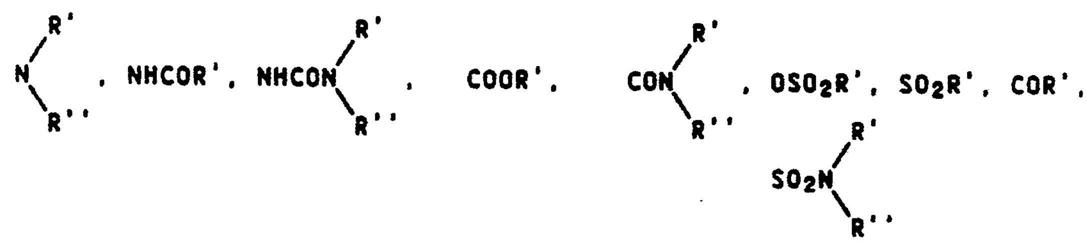

où $R^1$ et $R^2$ représentent, chacun indépendamment de l'autre, un atome d'hydrogène, un radical alkyle, alcoxy, alkylthio, cycloalkyle ou phényle à substitution alkylique, halogénée ou alcoxylique éventuelle et n représente les nombres 1 à 4,

à l'exception des composés dans lesquels $R^1$ et $R^2$ représentent le radical méthyle, X représente l'atome d'hydrogène et Y représente un atome d'hydrogène, d'halogène, un radical méthyle, éthyle, isopropyle, butyle tertiaire, méthoxy, trifluorométhoxy, nitro, amino, phényle, carboxyle, méthoxycarbonyle, cyano, acétylamino.

3. Procédé pour lutter contre des champignons, caractérisé en ce que l'on traite la terre, les semences, les plantes ou les matériaux menacés d'une attaque par des champignons, par une quantité efficace du point de vue fongicide d'un composé de la formule générale

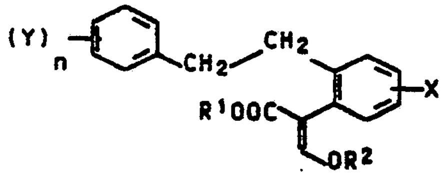

dans laquelle $R^1$ et $R^2$ représentent, indépendamment l'un de l'autre, un radical alkyle en $C_1$–$C_8$, X représente un atome d'hydrogène, un atome d'halogène, un radical alcoxy en $C_1$–$C_4$, trifluorométhyle, cyano ou nitro,
Y représente un atome d'hydrogène, un radical alkyle, halogénalkyle, alcoxyalkyle, cycloalkyle, aralkyle, aryle, aryloxy, un atome d'halogène, une chaîne $C_4H_4$ éventuellement substituée, condensée avec un reste de benzène, un radical alcoxy, halogénalcoxy, alkylthio, thiocyanato, cyano, $NO_2$

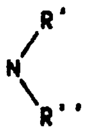 , NHCOR', 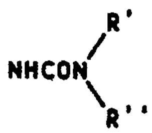 , COOR', 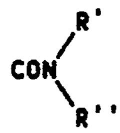 , $OSO_2R'$, $SO_2R'$, COR', 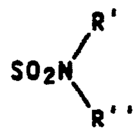

où $R^1$ et $R^2$ représentent, chacun indépendamment de l'autre, un atome d'hydrogène, un radical alkyle, alcoxy, alkylthio, cycloalkyle ou phényle à substitution alkylique, halogénée ou alcoxylique éventuelle et n représente les nombres 1 à 4,
à l'exception des composés dans lesquels $R^1$ et $R^2$ représentent le radical méthyle, X représente l'atome d'hydrogène et Y représente un atome d'hydrogène, d'halogène, un radical méthyle, éthyle, isopropyle, butyle tertiaire, méthoxy, trifluorométhoxy, nitro, amino, phényle, carboxyle, méthoxycarbonyle, cyano, acétylamino.